Europäisches Patentamt

(19) European Patent Office

Office européen des brevets

(11) Numéro de publication: **0 127 532**
**B1**

(12) **FASCICULE DE BREVET EUROPEEN**

(45) Date de publication du fascicule du brevet:
**26.08.87**

(21) Numéro de dépôt: **84401037.1**

(22) Date de dépôt: **18.05.84**

(51) Int. Cl.⁴: **G 21 C 17/10**

(54) Procédé de détection des défauts de répartition de la puissance du coeur d'un réacteur nucléaire à eau sous pression et dispositif de mise en oeuvre de ce procédé.

(30) Priorité: **19.05.83 FR 8308276**

(43) Date de publication de la demande:
**05.12.84 Bulletin 84/49**

(45) Mention de la délivrance du brevet:
**26.08.87 Bulletin 87/35**

(84) Etats contractants désignés:
**BE CH DE GB IT LI SE**

(56) Documents cités:
**DE - A - 2 657 686**
**FR - A - 2 307 341**
**FR - A - 2 331 864**
**FR - A - 2 372 495**

(73) Titulaire: **FRAMATOME ET CIE., Tour Fiat 1, Place de la Coupole, F-92400 Courbevoie (FR)**

(72) Inventeur: **Leroy, Jean, 91 avenue du Général Leclerc, F-91190 Gif-sur-Yvette (FR)**
Inventeur: **Ruiz, Pierre, 15 avenue Georges Clémenceau, F-93150 Blanc Mesnil (FR)**

(74) Mandataire: **Lavoix, Jean et al, c/o Cabinet Lavoix 2, Place D'Estienne D'Orves, F-75441 Paris Cedex 09 (FR)**

## Description

La présente invention a pour objet de détecter des défauts dans la répartition de puissance du coeur d'un réacteur nucléaire à eau sous pression.

La puissance d'un réacteur nucléaire à eau sous pression peut être réglée par différents moyens, parmi lesquels la commande de l'insertion ou de l'extraction des grappes de contrôle, c'est-à-dire de barres de commande en matériau absorbant les neutrons. Ces grappes de contrôle sont disposées parallèlement les unes aux autres à l'intérieur du coeur du réacteur, entre les éléments combustibles.

Il est très important, lors du fonctionnement du réacteur, de contrôler non seulement la puissance du réacteur mais également la répartition de cette puissance. En effet, une mauvaise répartition de la puissance peut porter atteinte à l'intégrité des éléments combustibles qui peuvent subir une dégradation irréversible sous l'effet de la chaleur qu'ils dégagent, si cette quantité de chaleur est trop importante pour être absorbée et entraînée par le fluide caloporteur ou si la circulation de fluide caloporteur est insuffisante. Les deux phénomènes que l'on veut surtout éviter sont d'une part la crise d'ébullition de l'eau sous pression au contact des éléments combustibles et d'autre part la fusion du combustible. La crise d'ébullition peut conduire à la formation d'un film de vapeur le long des éléments combustibles et l'eau sous pression, par un phénomène de caléfaction. Quant à la fusion du combustible, elle est due à un trop grand dégagement de puissance se traduisant par une élévation locale de température allant jusqu'au point de fusion du matériau; la fusion se produit lorsque la valeur de la puissance par unité de longueur ou puissance linéique dépasse un certain seuil.

Les deux phénomènes précités portant atteinte à l'intégrité des éléments combustibles peuvent être dus à une mauvaise position des grappes de contrôle: chute intempestive d'une grappe de contrôle qui se décroche de sa tige de commande; grappe non accrochée qui reste complètement insérée à la mise en service du réacteur; décalage d'une grappe par rapport aux grappes voisines.

On sait repérer la position des tiges de commande des grappes mais, dans le cas d'une grappe qui se décroche de sa tige de commande, par exemple, le repérage de la position des tiges de commande ne permettrait pas de détecter un défaut.

On connaît, par le FR-A-2 331 864, un procédé et un dispositif de détection de défauts de répartition de puissance du coeur d'un réacteur nucléaire; dans ce procédé on mesure un paramètre représentatif de la puissance du coeur en un nombre prédéterminé d'emplacements symétriques les uns des autres à la périphérie du coeur, on calcule la moyenne des valeurs mesurées du paramètre et on fait la somme des valeurs de mesure qui s'écartent de la moyenne dans un sens donné. La comparaison de cette somme à une valeur de consigne permet de déterminer une répartition de puissance anormale. Une telle méthode ne fournit cependant pas une indication très précise et très sûre dans le cas d'une anomalie dans le positionnement de certaines barres de commande.

C'est pourquoi le but de la présente invention est un procédé de détection des défauts de répartition de la puissance du coeur d'un réacteur nucléaire à eau sous pression dans lequel on mesure au moins un paramètre représentatif de la puissance du coeur, chaque paramètre étant mesuré en un nombre déterminé de points, procédé qui permette de détecter, de façon très sensible, tous les défauts de répartition de puissance du coeur, quelle que soit leur localisation dans le coeur et quelle que soit leur cause. Le but de l'invention est également de pouvoir détecter les défauts de façon très sûre, même lorsque certains capteurs sont, pour une raison quelconque, hors d'état de fonctionner.

Le procédé de détection des défauts de répartition de la puissance d'un réacteur nucléaire à eau sous pression selon l'invention est caractérisé par le fait que, pour chaque paramètre, on effectue la différence entre les deux valeurs extrêmes mesurées et le rapport de cette différence par la valeur mesurée la plus petite et on compare ce rapport à une valeur de consigne, un défaut étant détecté si ce rapport est supérieur à ladite valeur de consigne.

Afin d'obtenir une sensibilité encore plus grande, on peut calculer en outre la dérivée par rapport au temps dudit rapport et comparer cette dérivée à une valeur de consigne, un défaut étant détecté si cette dérivée est supérieure à ladite valeur de consigne.

De préférence, afin d'obtenir la sécurité optimale, les points de mesure de chaque paramètre sont répartis en plusieurs groupes, chaque point d'un groupe ayant dans chaque autre groupe un point homologue pour lequel la mesure dudit paramètre est redondante; on applique alors le procédé précédemment décrit à chacun des groupes et l'on déclenche des mesures de sécurité si un défaut est détecté dans au moins n groupes, n étant un nombre entier fixé à l'avance, inférieur au nombre total de groupes.

L'invention concerne également un dispositif de mise en œuvre du procédé précité pour la détection des défauts de répartition de la puissance dégagée par le cœur d'un réacteur nucléaire constitué par des assemblages combustibles placés côte à côte. Ce dispositif comporte, de manière connue, des moyens de mesure d'au moins un paramètre représentatif de la puissance du cœur disposés symétriquement à proximité de la périphérie du cœur. Selon l'invention, ces moyens de mesure sont constitués par au moins huit capteurs de mesure, chaque capteur étant associé à au moins deux capteurs homologues pour obtenir une mesure redondante et le dispositif comporte en outre des moyens de calcul du rapport de la différence entre les deux valeurs extrêmes mesurées par les capteurs à la valeur mesurée la plus petite et des moyens de comparaison de ce rapport à une valeur de consigne.

Dans un premier mode de réalisation du dispositif selon l'invention, les capteurs de mesure sont des chambres de mesure de flux neutronique hors cœur multi-étagées.

Dans un deuxième mode de réalisation de l'invention, les capteurs de mesure sont des capteurs de température.

Dans un troisième mode, préféré, les capteurs de mesure sont constitués de chambres de mesure de flux neutronique et de capteurs de température.

Dans ce cas, chaque chambre de mesure de flux est associée à trois autres chambres homologues, situées à des étages différents, pour lesquels la mesure est redondante et les capteurs de température sont situés chacun à la sortie d'un canal, c'est-à-dire d'un intervalle entre les assemblages combustibles du cœur, où ils sont associés à deux capteurs homologues pour lesquels la mesure est redondante.

Le dispositif selon l'invention comporte en outre de préférence, à proximité du centre du cœur, un capteur de mesure d'un paramètre représentatif de la puissance au centre du cœur, des moyens de comparaison de la valeur mesurée par ce capteur avec la moyenne des valeurs mesurées par les capteurs de mesure du même paramètre placés à proximité de la périphérie du cœur, et des moyens de correction de la valeur mesurée par le capteur situé à proximité du centre du cœur afin de tenir compte des écarts normaux entre le centre et la périphérie du cœur, ledit capteur de mesure étant associé à au moins deux capteurs homologues pour lesquels la mesure est redondante.

On va maintenant décrire, à titre d'exemple non limitatif, un mode de réalisation préféré de l'invention.

La figure 1 représente une vue en plan du cœur d'un réacteur nucléaire muni de capteurs de mesure de paramètres représentatifs de la puissance du cœur.

La figure 2 représente une coupe suivant AA de la figure 1.

La figure 3 représente le dispositif d'interprétation des signaux de mesure de flux neutronique mesurés par les chambres de mesure de flux des figures 1 et 2.

La figure 4 est une figure analogue à la figure 3 mais s'appliquant aux signaux de mesure de température.

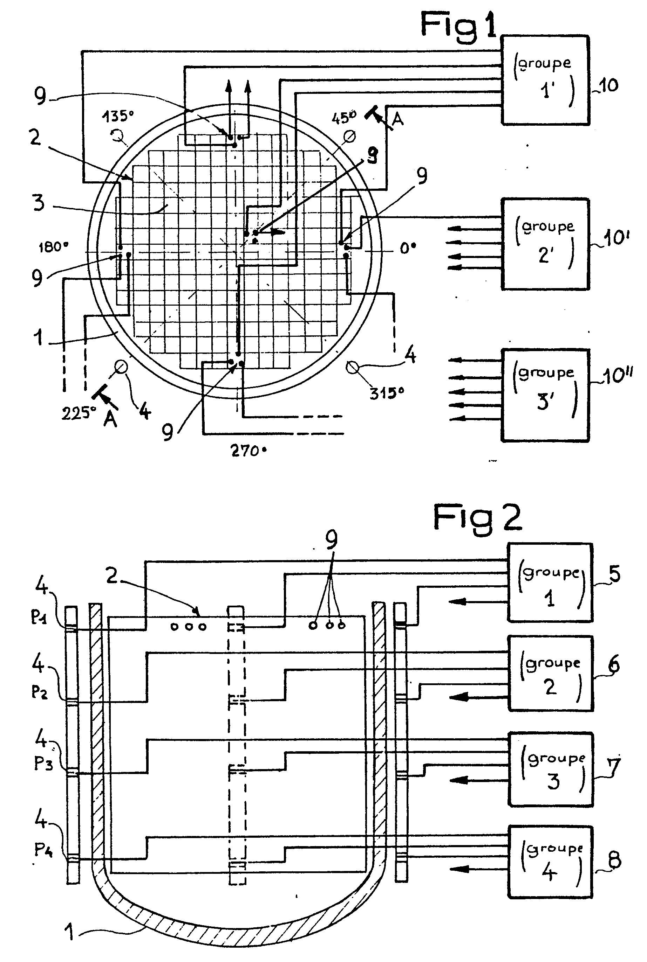

On se reportera tout d'abord aux figures 1 et 2.

Ces figures représentent schématiquement une cuve de réacteur 1 contenant un cœur 2 dans lequel on a dessiné un certain nombre de cases 3 (voir figure 1); ces cases contiennent, de façon classique, des éléments combustibles disposés parallèlement les uns aux autres, et certaines contiennent en outre des barres de commande en matériau absorbant les neutrons, disposées en grappes, et pouvant glisser dans des tubes-guides placés dans les éléments combustibles. Les éléments combustibles et les grappes n'ont pas été représentés, afin de ne pas surcharger les figures.

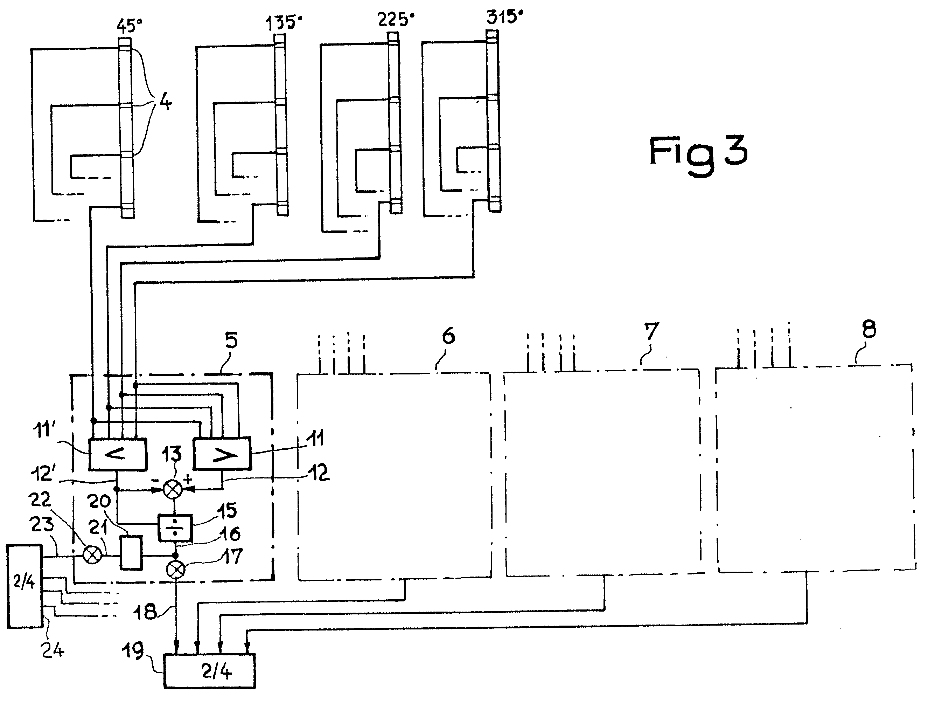

Des chambres de mesure de flux neutronique 4 sont disposées à l'extérieur du cœur, proches de sa périphérie, dans quatre plans parallèles différents, et dans chaque plan, aux extrémités de deux axes de symétrie du cœur. Ainsi, on voit clairement que dans le plan de la figure 1 se trouvent quatre chambres 4 situées à 45°, 135°, 225° et 315°. Les chambres 4 sont des chambres tout-à-fait classiques qui émettent un signal de flux neutronique, fonction de la puissance du cœur. Dans la suite, on appellera «groupe» l'ensemble des chambres de flux neutronique 4 situées dans un même plan. Ainsi, les quatre chambres représentées sur la figure 1 appartiennent au même groupe. On a représenté sur la figure 2 les plans $P_1$, $P_2$, $P_3$, $P_4$ comportant respectivement les chambres de flux neutronique 4 appartenant au groupe 1, au groupe 2, au groupe 3 ou au groupe 4. Les signaux émis par les chambres de flux neutronique 4 de chaque groupe entrent dans l'un des quatre dispositifs d'interprétation des signaux de chambres de flux neutronique 5, 6, 7 ou 8, suivant qu'ils appartiennent respectivement aux groupes 1, 2, 3 ou 4. Les dispositifs 5, 6, 7 ou 8 sont représentés plus en détail sur la figure 3 qui sera décrite plus bas.

Des capteurs de température 9 sont disposés par trois, dans le plan de la figure 1, à la périphérie du cœur, et près du centre du cœur. Ces capteurs 9 sont situés uniquement dans un plan, à la sortie des canaux dans lesquels circule le fluide primaire. Les valeurs mesurées par les capteurs 9 sont donc les valeurs de la température de sortie du fluide primaire en divers points de sortie de ce fluide et sont représentatives de la puissance du réacteur. (La température d'entrée du fluide primaire est supposée la même pour tous les canaux; une variation éventuelle de cette température entraîne donc une variation identique de toutes les valeurs mesurées par les capteurs 9 et n'intervient pas dans la détection des défauts de répartition de la puissance). Les capteurs 9 sont disposés sur deux axes perpendiculaires, situés dans le plan de la figure 1, à 0°, 90°, 180°, 270°, en ce qui concerne les capteurs périphériques, les trois autres capteurs étant situés à proximité de la grappe centrale. Les deux axes sur lesquels sont situés les capteurs périphériques constituent les bissectrices des angles formés par les axes sur lesquels sont situées les chambres de flux neutronique. Les capteurs de température 9 constituent trois groupes, 1', 2' et 3', chaque groupe étant constitué de cinq éléments situés respectivement à 0°, 90°, 180°, 270°, et à proximité du centre du cœur. Comme on peut considérer que la température mesurée est la même pour les trois capteurs situés à la sortie d'un même canal, chaque capteur d'un groupe possède donc un capteur de température homologue effectuant une mesure redondante dans les autres groupes, de la même façon que les quatre chambres de flux neutronique 4 situées l'une au-dessus de l'autre dans des étages différents effectuent des mesures de flux redondante.

Les signaux émis par les capteurs de température d'un même groupe entrent dans un dispositif d'interprétation des signaux 10, 10' ou 10". Ces dispositifs sont au nombre de trois, chaque dispositif correspondant à un groupe de capteurs 9. Sur la figure 2, les dispositifs 10, 10', 10" n'ont pas été représentés.

On se reportera maintenant à la figure 3 qui représente en détail le dispositif d'interprétation des signaux émis par les chambres neutroniques 4. On décrira l'interprétation des signaux du groupe 1 uniquement, l'interprétation des signaux des groupes 2, 3 et 4 étant analogue. Les signaux de mesure de flux d'un étage, c'est-à-dire les signaux des chambres 4 situées dans un même plan, à 45°, 135°, 225° et 315°, sont appliqués à l'entrée d'un dispositif d'interprétation, à savoir, pour le groupe 1 ici décrit, le dispositif 5. Les signaux entrent d'abord dans deux circuits sélecteurs 11 et 11', le premier circuit, 11, choisit le signal le plus grand et le second circuit, 11', choisit le signal le plus petit parmi les quatre signaux appliqués. Les deux signaux de sortie de ces circuits 11 et 11', à savoir 12 et 12', sont comparés en 13 et le signal 14 ainsi obtenu est divisé en 15 par le signal 12', à savoir le signal le plus petit. Le signal de sortie 16 est significatif d'une dissymétrie dans la répartition de puissance du cœur. Ce signal 16 est appliqué à un relais à seuil 17 qui, en fonction d'une valeur de consigne, délivre un signal logique, témoin d'un défaut de répartition de la puissance du cœur. Ce signal a été repéré par 18. Les signaux analogues aux signaux 18 issus des dispositifs 6, 7 et 8 correspondant aux groupes 2, 3 et 4, sont appliqués, en même temps que le signal 18 issu du dispositif 5 dans un dispositif 19 à logique majoritaire 2/4. Ainsi, si au moins deux des signaux tels que 18 dépassent un certain seuil, des mesures de sécurité sont déclenchées car l'on considère qu'un défaut est détecté.

Le signal 16 est également appliqué à un module 20 permettant d'obtenir le signal 21 représentant la dérivée temporelle du signal 16. Ce signal 21 est appliqué à un relais à seuil 22 qui le compare à une valeur de consigne interne. Le signal issu de ce relais 22 a été repéré par 23 et s'applique à un dispositif 24 de logique majoritaire 2/4. Dans ce dispositif 24 sont introduits également les signaux issus des dispositifs 6, 7 et 8 analogues au signal 23 mais concernant les groupes 2, 3 et 4. Des mesures de sécurité sont prises quand deux au moins des signaux 23 dépassent un certain seuil. On détecte ainsi les défauts fugitifs de répartition de puissance du cœur correspondant à un déséquilibre transitoire de celui-ci.

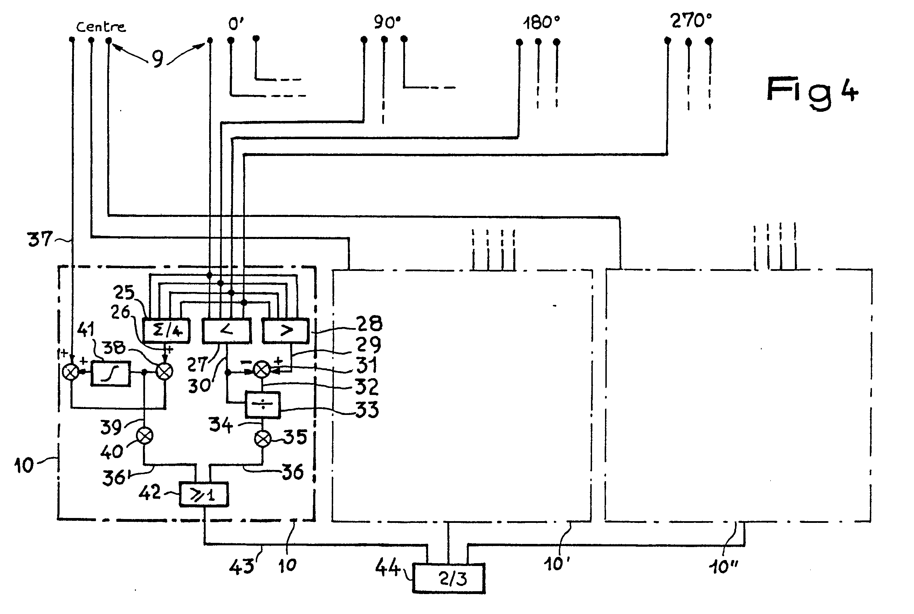

On se reportera maintenant à la figure 4 qui décrit les dispositifs 10, 10' et 10" d'interprétation des signaux émis par les capteurs de température 9. De la même façon que précédemment, on ne décrira que le dispositif 10 correspondant au groupe 1', les dispositifs 10' et 10" correspondant aux deux autres groupes étant analogues.

Les signaux de mesure des thermocouples périphériques, c'est-à-dire situés sur les axes 0°, 90°, 180° et 270°, sont appliqués à un circuit 25 qui détermine un signal de valeur moyenne 26, d'une part, et à deux circuits sélecteurs 27 et 28 qui déterminent le signal le plus grand 29 et le signal le plus petit 30, d'autre part.

L'utilisation du signal 26 sera décrite plus loin. Les signaux 29 et 30 sont comparés en 31 et le signal 32 issu du comparateur 31 est divisé en 33 par le signal 30, à savoir le signal le plus petit. Le signal 34 ainsi obtenu est significatif d'une dissymétrie dans la répartition de puissance du cœur. Ce signal 34 est appliqué à un relais à seuil 35 qui, en fonction d'une valeur de consigne, délivre un signal logique 36.

Le signal de température moyenne 26 des thermocouples périphériques est comparé en permanence au signal de mesure 37 des thermocouples centraux. Le comparateur de ces deux signaux est repéré par 38. Le signal de sortie 39 de ce comparateur, représente l'écart entre la température du centre et la température moyenne de la périphérie du cœur, et est appliqué à un relais à seuil 40 permettant d'obtenir un signal logique significatif d'une détection de dissymétrie entre le centre et la périphérie du cœur. Un intégrateur 41 permet de recaler en permanence le signal 37 émis par le thermocouple central afin de tenir compte des écarts normaux de puissance entre le centre du cœur et la périphérie du cœur.

Les signaux 36 et 36' issus des relais à seuil 35 et 40 sont appliqués à un dispositif logique 42 dont le signal de sortie 43 est nul si les deux signaux 36 et 36' sont nuls, mais est égal à un si au moins l'un des deux signaux 36 ou 36' est égal à un c'est-à-dire si un défaut est détecté à la périphérie du cœur ou entre le centre et la périphérie du cœur.

Le signal de sortie 43 est appliqué ainsi que les signaux analogues issus des groupes 2 et 3 à un dispositif 44 à logique majoritaire 2/3. Ainsi des mesures de sécurité sont prises si un défaut de répartition de puissance est détecté dans au moins deux sur trois des groupes de capteurs de température.

Ainsi, l'invention permet de détecter un défaut de répartition de la puissance du cœur quel que soit l'endroit où est situé ce défaut, dans l'un des quatre quadrants du cœur, ou sur l'un des quatre axes représentés sur la figure 1 ou encore au centre du cœur. l'invention permet de détecter non seulement les défauts permanents mais aussi, au moyen du calcul de la dérivée, les défauts fugitifs pendant les déséquilibres transitoires. Le mode de calcul utilisé permet une détection très sensible des défauts. En outre le fait d'utiliser des mesures redondantes apporte une grande sécurité et permet aussi de réparer sans avoir à arrêter le fonctionnement du cœur, l'un des groupes de capteurs éventuellement défectueux.

Bien entendu, l'invention ne se limite pas au mode de réalisation qui a été décrit uniquement à titre d'exemple mais elle couvre également d'autres modes qui n'en différeraient que par des détails, par des variantes d'exécution ou par l'utilisation de moyens équivalents.

Ainsi, le choix et l'emplacement des capteurs de mesure peuvent varier en fonction de la structure géométrique du cœur (par exemple carrée ou hexagonale), du type de symétrie existant dans les groupes de grappes de contrôle manœuvrées si-

multanément, ainsi que des contraintes liées éventuellement aux autres fonctions de ces détecteurs dans le système de protection et de pilotage du réacteur.

On aurait pu ainsi utiliser des chambres de flux neutronique plutôt que des capteurs de température pour contrôler le centre du cœur. Le dispositif de l'interprétation des signaux aurait été semblable au dispositif décrit pour l'interprétation des signaux des capteurs de mesure de température centraux.

On aurait pu aussi utiliser uniquement des capteurs de mesure de température ou uniquement des chambres de mesure de flux neutronique.

Il aurait été également possible de placer des capteurs à l'intérieur du cœur et non pas seulement à la périphérie. Il aurait alors fallu prévoir des dispositifs de recalage pour tenir compte des écarts de température qui existent de façon normale à l'intérieur du cœur, la température diminuant du centre vers la périphérie du cœur.

D'autre part, les dispositifs 19 et 24 pourraient être conçus différemment, par exemple de logique majoritaire 3/4.

Le nombre de groupes de capteurs de température pourrait également être différent, par exemple 4, le dispositif 44 étant alors de logique majoritaire 2/4 ou 3/4.

**Revendications**

1. Procédé de détection des défauts de répartition de la puissance du cœur d'un réacteur nucléaire à eau sous pression, dans lequel on mesure au moins un paramètre représentatif de la puissance du cœur, chaque paramètre étant mesuré en un nombre déterminé de points, caractérisé par le fait que, pour chaque paramètre, on effectue la différence entre les deux valeurs extrêmes mesurées (12 et 12'; 29 et 30) et le rapport (16; 34) de cette différence par la valeur mesurée la plus petite (12'; 30) et l'on compare ce rapport (16; 34) à une valeur de consigne, un défaut étant détecté si ce rapport (16; 34) est supérieur à ladite valeur de consigne.

2. Procédé de détection selon revendication 1, caractérisé par le fait que l'on calcule en outre la dérivée (21) par rapport au temps dudit rapport (16) et que l'on compare cette dérivée (21) à une valeur de consigne, un défaut étant détecté si cette dérivée est supérieure à ladite valeur de consigne.

3. Procédé de détection selon l'une des revendications 1 ou 2, caractérisé par le fait que les points de mesure (4; 9) de chaque paramètre sont répartis en plusieurs groupes (groupes 1, 2, 3, 4; groupes 1', 2', 3'), chaque point d'un groupe ayant dans chaque autre groupe un point (4; 9) homologue, pour lequel la mesure dudit paramètre est redondante, que l'on applique le procédé selon la revendication 1 ou 2 à chacun des groupes (groupes 1, 2, 3, 4; groupes 1', 2', 3') et que l'on déclenche des mesures de sécurité si un défaut est détecté dans au moins n groupes, n étant un nombre entier fixé à l'avance, inférieur au nombre total de groupes.

4. Dispositif de mise en œuvre du procédé selon l'une quelconque des revendications 1 à 3, pour la détection des défauts de répartition de la puissance dégagée par le cœur d'un réacteur nucléaire constitué par des assemblages combustibles placés côte à côte, comportant, de manière connue, des moyens de mesure d'au moins un paramètre représentatif de la puissance du cœur, disposés symétriquement par rapport à la périphérie du cœur, caractérisé par le fait que les moyens de mesure sont constitués par au moins huit capteurs de mesure (4, 9) chaque capteur (4, 9) étant associé à au moins deux capteurs homologues pour obtenir une mesure redondante et qu'il comporte en outre des moyens de calcul (5, 6, 7, 8, 10, 10', 10'') du rapport de la différence entre les deux valeurs extrêmes mesurées par les capteurs à la valeur mesurée la plus petite et de comparaison de ce rapport à une valeur de consigne.

5. Dispositif selon la revendication 4, caractérisé par le fait que les capteurs de mesure sont des chambres de mesure (4) de flux neutronique hors cœur multi-étagées.

6. Dispositif selon la revendication 4, caractérisé par le fait que les capteurs de mesure sont des capteurs de température (9).

7. Dispositif selon la revendication 4, caractérisé par le fait que les capteurs de mesure sont constitués de chambres de mesure de flux neutronique (4) et de capteurs de température (9).

8. Dispositif selon la revendication 7, caractérisé par le fait que chaque chambre de mesure de flux neutronique (4) est associée à trois autres chambres homologues situées à des étages différents, pour lesquelles la mesure est redondante, et que les capteurs de température (9) sont situés chacun à la sortie d'un canal et sont associés à deux capteurs homologues pour lesquels la mesure est redondante.

9. Dispositif selon l'une quelconque des revendications 4 à 8, caractérisé par le fait qu'il comporte en outre, à proximité du centre du cœur, un capteur de mesure (9) d'un paramètre représentatif de la puissance au centre du cœur, des moyens de comparaison (38) de la valeur (37) mesurée par ce capteur (9) avec la moyenne (26) des valeurs mesurées par les capteurs de mesure (9) du même paramètre placés à proximité de la périphérie du cœur et des moyens de correction (41) de la valeur (37) mesurée par ce capteur (9) pour tenir compte des écarts normaux de puissance entre le centre et la périphérie du cœur, ledit capteur de mesure (9) étant associé à au moins deux capteurs homologues (9) pour lesquels la mesure est redondante.

**Patentansprüche**

1. Verfahren zum Erfassen von Fehlern in der Leistungsverteilung des Kerns eines Druckwasserkernreaktors, bei dem mindestens ein für die Leistung des Kerns repräsentativer Parameter gemessen wird und jeder Parameter an einer bestimmten Anzahl von Stellen gemessen wird, dadurch gekennzeichnet, dass für jeden Parameter die Differenz zwischen zwei gemessenen Extremwerten (12 und 12'; 29 und 30) und das Verhältnis (16; 34) dieser Differenz zu dem kleineren gemessenen Wert (12'; 30) bestimmt werden und dieses Verhältnis (16; 34) mit einem Bezugswert verglichen wird, wobei ein Fehler erfasst wird, wenn dieses Verhältnis (16; 34) grösser als der Bezugswert ist.

2. Verfahren nach Anspruch 1, dadurch gekennzeichnet, dass ferner die zeitliche Ableitung (21) des Verhältniswertes (16) berechnet wird und dass dieses Verhältnis (21) mit einem Bezugswert verglichen wird, wobei ein Fehler erfasst wird, wenn diese Ableitung grösser als der Bezugswert ist.

3. Verfahren nach einem der Ansprüche 1 oder 2, dadurch gekennzeichnet, dass die Messpunkte (4; 9) jedes Parameters in mehreren Gruppen (Gruppen 1, 2, 3, 4; Gruppen 1', 2', 3') verteilt sind, wobei jeder Punkt einer Gruppe in jeder der anderen Gruppen einen entsprechenden Punkt (4; 9) hat, für den die Messung des besagten Parameters redundant ist, dass das Verfahren nach Anspruch 1 oder 2 bei jeder der Gruppen (Gruppen 1, 2, 3, 4; Gruppen 1', 2', 3') durchgeführt wird und dadurch, dass Sicherheitsmassnahmen ausgelöst werden, wenn ein Fehler in mindestens N-Gruppen erfasst wird, wobei N eine vorbestimmte ganze Zahl ist, die kleiner als die Gesamtanzahl der Gruppen ist.

4. Vorrichtung zur Durchführung der Verfahrens nach einem der Ansrpüche 1–3 zur Erfassung von Fehlern in der Verteilung der Leistung, die durch den Kern eines Kernreaktors freigesetzt wird, der aus Seite an Seite angeordneten Brennelementen besteht, die in bekannter Weise Organe zur Messung mindestens eines für die Leistung des Kerns repräsentativen Parameters aufweisen, die symmetrisch bezüglich der Peripherie des Kerns angeordnet sind, dadurch gekennzeichnet, dass die Messorgane aus mindestens acht Messfühlern (4, 9) bestehen und jeder Messfühler (4, 9) mit mindestens zwei homologen Messfühlern zum Erhalten einer redundanten Messung in Verbindung steht und ausserdem Rechenorgane (5, 6, 7, 8, 10, 10', 10'') des Verhältnisses der Differenz zwischen zwei Extremwerten, die von den Messfühlern gemessen werden zu dem kleinsten Messwert und Organe zum Vergleichen dieses Verhältnisses mit einem Bezugswert aufweisen.

5. Vorrichtung nach Anspruch 4, dadurch gekennzeichnet, dass die Messfühler Neutronenflussmesskammern (4) ausserhalb des mehretagigem Reaktorkerns sind.

6. Vorrichtung nach Anspruch 4, dadurch gekennzeichnet, dass die Messfühler Temperaturmessfühler (9) sind.

7. Vorrichtung nach Anspruch 4, dadurch gekennzeichnet, dass die Messfühler aus Neutronenflussmesskammern (4) und aus Temperaturfühlern (9) bestehen.

8. Vorrichtung nach Anspruch 7, dadurch gekennzeichnet, dass jede Neutronenflussmesskammer (4) mit drei homologen Messkammern verbunden ist, die in verschiedenen Etagen angeordnet sind, für die die Messung redundant ist, und dadurch, dass die Temperaturmessfühler (9) jeweils am Ausgang des Kanals angeordnet und jeweils mit zwei homologen Messfühlern verbunden sind, für die die Messung redundant ist.

9. Vorrichtung nach einem der Ansprüche 4–8, dadurch gekennzeichnet, dass sie ausserdem in der Nähe des Zentrums des Kerns einen Messfühler (9) eines Parameters aufweist, der für die Leistung des Zentrums des Kerns repräsentativ ist, dass sie ferner Vergleichsorgane (38) des durch den Fühler (9) gemessenen Wertes (37) mit dem Mittel (26) der mit den Messfühlern (9) desselben Parameters gemessen worden sind, die in der Nähe der Peripherie des Kerns angeordnet sind und dass sie ferner Korrekturorgane (41) des Wertes (37), der durch den Messfühler (9) gemessen worden ist, aufweist, um den normalen Leistungsunterschieden zwischen dem Zentrum und der Peripherie des Kernes Rechnung zu tragen, wobei dieser Messfühler (9) mit mindestens zwei homologen Messfühlern (9) verbunden ist, damit diese Messung redundant ist.

**Claims**

1. Process for detecting faults in the distribution of the core power of a pressurized water nuclear reactor, wherein at least one parameter representing the power of the core is measured, each parameter being measured at a determined number of points, characterized by the fact that for each parameter, the difference between the two extreme measured values (12 and 12'; 29 and 30) and the ratio (16; 34) of this difference to the smallest measured value (12'; 30) are determined, and the ratio (16; 34) is compared to a set value, a fault being detected if this ratio (16; 34) is greater than the said set value.

2. The detecting process as claimed in claim 1, characterized by the fact that, in addition, the derivative (21) with respect to time of the said ratio (16) is calculated and this derivative (21) is compared to a set value, a fault being detected if this derivative is greater than the said set value.

3. The detecting process as claimed in one of claims 1 or 2, characterized by the fact that the points (4; 9) for measuring each parameter are distributed in several groups (groups 1, 2, 3 and 4; groups 1', 2' and 3'), each point of a group having in each other group a homologous point (4; 9) for which the measurement of the said parameter is redundant, that the process as claimed in claim 1 or 2 is applied to each of the groups (groups 1, 2, 3 and 4; groups 1', 2', and 3') and that safety measures are triggered if a fault is detected in at least n groups, n being a whole number fixed in advance, and smaller than the total number of groups.

4. Device for making use of the process as claimed in any one of claims 1 to 3, for detecting faults in the distribution of the core power of a nuclear reactor comprised of fuel assemblies placed side by side, comprising in a known way, means for measuring at least one parameter representing the core power, symetrically disposed in respect with the core periphery characterized by the fact that the measuring means are comprised of at least eight measuring sensors (4, 9), each sensor (4, 9) being associated with at least two homologous sensors for which the measurement is redundant, and that it further comprises means for calculating (5, 6, 7 and 8; 10, 10' and 10'') the ratio of the difference between the two extreme values measured by the sensor to the smaller one and for comparison of the said ratio set value.

5. The device as claimed in claim 4, characterized by the fact that the measuring sensors are multi-level neutron flux measuring ex-core chambers (4).

6. The device as claimed in claim 4, characterized by the fact that the measuring sensors are temperature sensors (9).

7.The device as claimed in claim 5, characterized by the fact that the measuring sensors consist of neutron flux measurement chambers (4) and temperature sensors (9).

8. The device as claimed in claim 7, characterized by the fact that each neutron flux measuring chamber (4) is associated with three other homologous chambers situated at different levels, for which the measurement is redundant, and wherein the temperature sensors (9) are each situated at the exit of a channel and are associated with two homologous sensors for which the measurement is redundant.

9. The device as claimed in any one of claims 4 to 8, which moreover comprises, close to the center of the core, a sensor (9) for measuring a parameter representing the power at the center of the core, means for comparing (38) the value (37) measured by this sensor (9) with the mean (26) of the values measured by the sensors for measuring (9) the same parameter which are placed close to the periphery of the core and means for correcting (41) the value (37) measured by this sensor (9) to take account of the normal differences in power between the center and the periphery of the core, the said measuring sensor (9) being associated with at least two homologous sensors (9) for which the measurement is redundant.

# Fig 1

groupe 1'  10

groupe 2'  10'

groupe 3'  10''

9

135°

45° A

9

2

3

180°

9

9

0°

1

4

225° A 4

9

315°

270°

# Fig 2

9

4 2

P1

groupe 1  5

4

P2

groupe 2  6

4

P3

groupe 3  7

4

P4

groupe 4  8

1

Fig 3

Fig 4